# EUROPEAN PATENT SPECIFICATION

(11) **EP 3 509 664 B1**
(45) Date of publication and mention of the grant of the patent: **03.08.2022**
(21) Application number: 17866455.3
(22) Date of filing: 09.10.2017
(51) Int. Cl.: A61M 3/02, A61B 18/14, F04B 43/00, F04B 43/12

(54) **INTERFACE TUBING FOR PERISTALTIC PUMP**
VERBINDUNGSSCHLAUCH FÜR PERISTALTIKPUMPE
TUBULURE D'INTERFACE POUR POMPE PÉRISTALTIQUE

(30) Priority: 02.11.2016 US 201662416204 P
(43) Date of publication of application: 17.07.2019
(73) Proprietor: St. Jude Medical, Cardiology Division, Inc., St. Paul, MN 55117 (US)
(72) Inventor: SCHMIDT, Brian, Cottage Grove Minnesota 55016 (US); LEACH, Coleman, St. Paul Minnesota 55101 (US); KLOOSTERBOER, Michael, Minneapolis Minnesota 55413 (US); SWANSON, Lawrence D., White Bear Lake Minnesota 55110 (US); PARSONAGE, Edward E., St. Paul Minnesota 55116 (US)
(74) Representative: Kramer Barske Schmidtchen Patentanwälte PartG mbB
(86) International application number: PCT/US2017/055717
(87) International publication number: WO 2018/085000

(56) References cited:
- WO-A1-2016/076363
- WO-A2-2008/109863
- GB-A- 2 234 800
- GB-A- 2 476 698
- US-A1- 2007 224 063
- US-A1- 2012 282 126
- US-A1- 2016 185 549
- US-B2- 7 754 277

## Description

### CROSS-REFERENCE TO RELATED APPLICATIONS

This application is related to United States provisional application no. 62/416,204, filed 2 November 2016.

### BACKGROUND

The instant disclosure relates to irrigated electrophysiology catheters. In particular, the present disclosure relates to devices and methods for reducing electrical noise in an irrigated electrophysiology catheter system.

Catheters are used for an ever growing number of medical procedures. To name just a few examples, catheters are used for diagnostic, therapeutic, and ablative procedures. Typically, the physician manipulates the catheter through the patient's vasculature to the intended site, such as a site within the patient's heart. The catheter typically carries one or more electrodes (in the case of so-called "electrophysiology catheters") or other diagnostic or therapeutic devices, which can be used for ablation, diagnosis, cardiac mapping, or the like.

Irrigated electrophysiology catheters are also known. An irrigated electrophysiology catheter is an electrophysiology catheter that is equipped to deliver an irrigation fluid, such as saline, to a location proximate the electrodes. The irrigation fluid serves, for example, to cool the electrodes or to disperse body fluids therefrom, to cool or bathe surrounding tissue, and/or to couple the electrodes to the tissue surface in the case of relatively highly conductive fluid(s).

In many irrigated electrophysiology catheters, a peristaltic pump is used to deliver the irrigation fluid. Typical peristaltic pumps operate by rotating a number of rollers mounted on a rotor to periodically compress an irrigation tube between the rollers and a pump housing or clamp, which forces the irrigation fluid through the irrigation tube. A peristaltic pump is, for example, known from US 2007/0224063 A1. An anti-static tubing is known from GB 2 234 800A. An anti-static liner tube is known from GB 2 476 698 A. An antistatic tool is known from WO 2016/076363 A1. Further, triboelectric charging of plastics is discussed in the PhD thesis "Triboelektrische Aufladung von Kunststoffen", Technical University Bergakademie of Freiberg, written by Dipl.-Ing. Ernö Németh, 2003 and in "Triboelectricity in Polymers", Henniker, Nature, 3 November 1962.

It is known, however, that peristaltic pumps may generate electrical noise that can undesirably couple to an electrogram signal measured by the electrodes on the irrigated electrophysiology catheter, to an electroanatomical visualization, localization and/or position system coupled to a subject, and/or to other diagnostic or therapeutic equipment. This noise is referred to herein as the "noise signal." The components of the noise signal include electrostatic discharge ("ESD").

### BRIEF SUMMARY

The invention is defined by the appended claims. Disclosed herein is an irrigation system for use with an irrigated electrophysiology catheter, including: a peristaltic pump; and a pump interface tubing for use in a peristaltic pump that includes: a tubular core having an outer surface; and a treatment on the outer surface of the tubular core, wherein the treatment reduces static charge buildup on the tubular core during operation of the peristaltic pump. The tubular core includes a first material and the treatment includes a layer of a second material different from the first material formed about the tubular core. Alternatively, the treatment can include a layer of the first material mixed with an ESD-preventing additive formed about the tubular core. The treatment can extend along any or all of the length of the outer surface of the tubular core.

The second material is closer to zero on the triboelectric scale than the first material. For example, the second material can be a nitrile, a heat shrink material, or a cotton fiber. Additionally, the treatment can include an anti-static spray treatment (not forming part of the claimed invention).

In embodiments of the disclosure, the treatment can also resist abrasion of the tubular core during operation of the peristaltic pump.

The pump interface tubing can also include first and second keys at opposite ends thereof. The keys can be used to interconnect the pump interface tubing to an irrigation tube.

The treatment on the outer surface of the tubular core reduces a noise signal resulting from operation of the peristaltic pump.

Further, the treatment reduces static buildup on the pump interface tubing resulting from operation of the peristaltic pump.

According to another aspect of the disclosure, a method of manufacturing a reduced-noise pump interface tubing for use in a peristaltic pump of an irrigation system for use with an irrigated electrophysiology catheter, includes forming a noise-reduction layer about a tubular core.

The noise-reduction layer can be formed by forming a nitrile layer about the tubular core, such as by dip-coating the nitrile layer about the tubular core.

The noise-reduction layer can also be formed by co-extruding the tubular core from a first material and the noise-reduction from a second material different from the first material.

A noise-reduction layer can be formed by spraying the tubular core with an anti-static spray (not forming part of the claimed invention).

In further embodiments, the noise-reduction layer can be formed by forming a cotton fiber layer about the tubular core.

In still further embodiments, the noise-reduction layer can be formed by forming a heat shrink layer about the tubular core.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 depicts a peristaltic pump.
Figure 2 is a representative electrostatic discharge noise signal.
Figure 3 is an illustrative transverse cross-section of a pump interface tubing that depicts the tubular core and the noise reduction layer disclosed herein.
Figure 4 is a representative bipolar electrogram according to aspects of the instant disclosure that utilize a nitrile noise reduction layer.
Figure 5 is a representative bipolar electrogram according to aspects of the instant disclosure that utilize a cotton fiber noise reduction layer.
Figure 6 is a representative bipolar electrogram according to aspects of the instant disclosure that utilize an acrylated olefin heat shrink noise reduction layer.
Figure 7 is a representative bipolar electrogram according to aspects of the instant disclosure that utilize a co-extruded non-DEHP noise reduction layer.
Figure 8A is a representative bipolar electrogram according to aspects of the instant disclosure that utilize a general purpose staticide noise reduction layer.
Figure 8B is a representative bipolar electrogram according to aspects of the instant disclosure that utilize a licron crystal staticide noise reduction layer.

### DETAILED DESCRIPTION

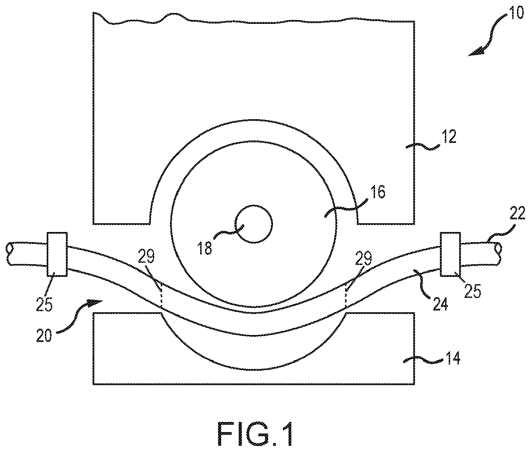

Figure 1 depicts a peristaltic pump 10, such as the Cool Point^{™} Irrigation Pump of St. Jude Medical, Inc. The configuration and operation of peristaltic pump 10 will be familiar to those of ordinary skill in the art *(see, e.g.,* United States patent application publication no. 2007/0224063 ), such that a detailed explanation thereof is not necessary herein. Instead, only those features of peristaltic pump 10 pertinent to understanding the present disclosure will be described below.

Peristaltic pump 10 generally includes a housing 12, a clamp 14, and a rotor 16. Rotor 16 includes a plurality of rollers spaced about the circumference of rotor 16 and is mounted to rotate about an axle 18.

A tubing channel 20 is defined between clamp 14 and rotor 16. Tubing channel 20 accommodates an irrigation tube 22. One end of irrigation tube 22 can be coupled to a suitable reservoir of irrigation fluid, while the opposite end of irrigation tube 22 can be coupled to an irrigated electrophysiology catheter. Thus, when in operation, peristaltic pump 10 moves irrigation fluid from the reservoir into the electrophysiology catheter, where it moves through one or more irrigation lumens and exits via one or more irrigation ports.

A portion of irrigation tube 22, referred to herein as the pump interface tubing 24, is positioned between clamp 14 and rotor 16, and is interconnected to the remainder of irrigation tube 22 by keys 25. One of ordinary skill in the art will appreciate that, as rotor 16 turns, the rollers will periodically (if evenly spaced about the circumference of rotor 16) impinge upon pump interface tubing 24, pushing pump interface tubing 24 against clamp 14 and forcing fluid through irrigation tube 22 to provide a pulsatile flow of irrigation fluid to the electrophysiology catheter.

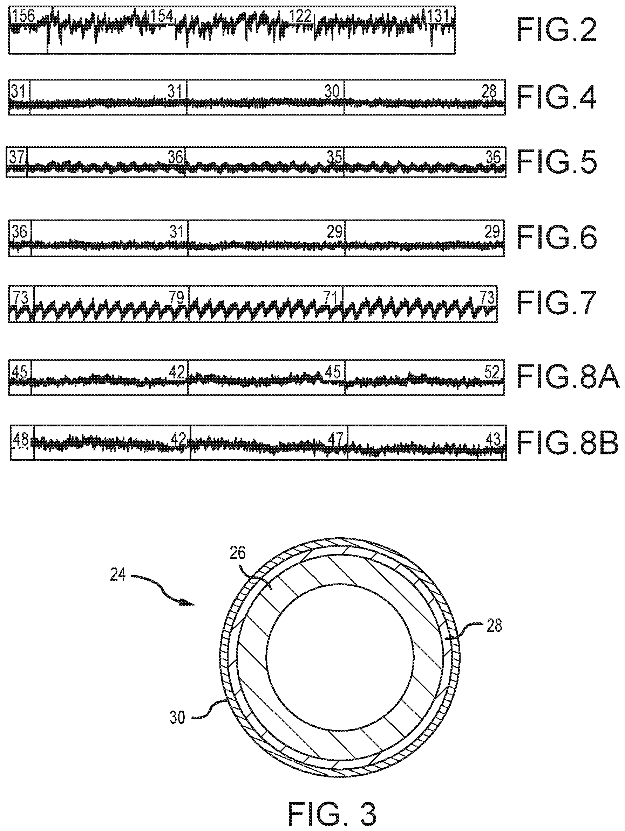

As described above, operation of peristaltic pump 10 can create a noise signal due, *inter alia,* to ESD. The noise signal can undesirably couple to an electrical signal of interest *(e.g.,* a bipolar electrogram measured using intracardiac electrodes). Figure 2 shows an illustrative ESD noise signal.

The instant disclosure provides various modifications and improvements to pump interface tubing 24 that desirably reduce or eliminate the noise signal. Several embodiments of the disclosure are illustrated schematically in Figure 3, which is a transverse cross-section of pump interface tubing 24 according to aspects disclosed herein.

As shown in Figure 3, pump interface tubing 24 includes a flexible, polymeric, and tubular core 26. Tubular core 26 can be made of a non-DEHP tubing, such as Tygon^{®} ND-100-65 from Saint-Gobain Performance Plastics, which uses TOTM as a plasticizer. The use of non-DEHP materials for tubular core 26 is desirable in that it preserves biocompatibility, though other biocompatible materials can be used for tubular core 26 without departing from the scope of the instant teachings.

Figure 3 also shows a treatment 28 on the outer surface of tubular core 26. As discussed in greater detail below, treatment 28 reduces static charge buildup on tubular core 26 during operation of peristaltic pump 10, and thus reduces the noise signal resulting from operation of peristaltic pump 10. Thus, treatment 28 is also referred to herein as a "noise-reduction layer."

As shown in Figure 3, it is desirable for treatment 28 to cover the complete circumference of tubular core 26. It is also desirable for treatment 28 to be sufficiently thick *(e.g.,* in the radial direction in Figure 3) that it is not compromised during operation of peristaltic pump 10 in a manner that exposes tubular core 26 to rotor 16. In this regard, in embodiments of the disclosure, an additional layer 30 of protective material can be applied over treatment 28 in order to enhance the durability of treatment 28. Layer 30 can include, by way of example only, wax, zinc stearate, erucamide, and other abrasion-resistant materials. The material used in layer 30 can be selected so as not to impair the ability of treatment 28 to reduce static charge buildup on tubular core 26 during operation of peristaltic pump 10.

In embodiments of the disclosure, treatment 28 is applied to the entire length of pump interface tubing 24 *(e.g.,* the entire length between keys 25 in Figure 1). In other embodiments, treatment 28 is applied only to the portion of pump interface tubing 24 that is proximate rotor 16 *(e.g.,* a region, about 3 inches long, between dotted lines 29 in Figure 1).

According to aspects of the disclosure, treatment 28 is a layer of a material that differs from the material of tubular core 26 *(e.g.,* it is other than Tygon^{®} ND-100-65). For example, treatment 28 can be a layer of a nitrile formed about tubular core 26, for example by dip coating or by otherwise securing a layer of a nitrile rubber to tubular core 26. Figure 4 depicts a representative electrogram signal resulting from the use of a nitrile layer for treatment 28.

Desirably, the electrogram signal of Figure 4 is largely free of noise signals. The inventors believe that this advantage can be attributed, at least in part, to nitrile rubber (+3) being closer to 0 on the triboelectric scale than is the material of tubular core 26 (often around -70).

In still further aspects of the disclosure, treatment 28 is a layer of a cotton fiber. Cotton fiber (+5) is also closer to 0 on the triboelectric scale than is the material of tubular core 26 (often around -70). Figure 5 depicts a representative electrogram signal resulting from the use of a cotton fiber layer for treatment 28.

In other aspects of the disclosure, treatment 28 is a layer of heat shrink material, such as an acrylated olefin. Figure 6 depicts a representative electrogram signal resulting from the use of an acrylated olefin heat shrink material for treatment 28.

Treatment 28 can also be co-extruded with tubular core 26. As those of ordinary skill in the art will appreciate, co-extrusion is a process by which two or more materials are pressed through the same die to produce a single piece. Thus, treatment 28 can be a different non-DEHP material from tubular core 26, such as Tygon^{®} E-LFL tubing, also from Saint-Gobain Performance Plastics. It is contemplated that tubular core 26 provides structural support to facilitate irrigant delivery, while treatment 28 provides an improvement in the noise signal relative to tubular core 26 alone, such as shown in the representative electrogram signal of Figure 7, which depicts a representative electrogram signal resulting from the use of a co-extruded Tygon^{®} E-LFL layer for treatment 28.

In other embodiments, the co-extruded material in treatment 28 includes an ESD-preventing additive mixed with a non-DEHP material, which non-DEHP material can be the same or different as the non-DEHP material of tubular core 26.

It is also contemplated that a treatment can be an anti-static spray applied to the outer surface of tubular core 26. Figure 8A shows a representative electrogram signal resulting from the use of a general-purpose staticide, while Figure 8B shows a representative electrogram signal resulting from the use of a licron crystal staticide.

Although several embodiments of this invention have been described above with a certain degree of particularity, those skilled in the art could make numerous alterations to the disclosed embodiments

For example, the teachings of the instant disclosure can be combined with those in United States patent application publication no. 2012/0165735 to achieve still further improvements in the minimization and/or elimination of noise signals.

As another example, although Figure 3 depicts treatment 28 and layer 30 as two discrete layers, they could also be implemented as a single layer that incorporates both materials, such as by impregnating or blending the noise-reducing material of treatment 28 with the abrasion-resistant material of layer 30. That is, in embodiments of the disclosure, the protective material (e.g., wax, zinc stearate, erucamide, or the like) can be blended with the material of treatment 28 (e.g., nitrile).

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting.

## Claims

1. An irrigation system for use with an irrigated electrophysiology catheter, comprising:
a peristaltic pump (10); and
a pump interface tubing (24) for use in a peristaltic pump (10), the pump interface tubing (24) comprising:
a tubular core (26) having an outer surface; and
a treatment (28) on the outer surface of the tubular core (26), wherein the treatment (28) reduces static charge buildup on the tubular core (28) during operation of the peristaltic pump (10),
wherein the tubular core (26) comprises a first material and wherein the treatment (28) comprises a layer of a second material different from the first material formed about the tubular core (26), and wherein the second material is closer to zero on the triboelectric scale than the first material.

2. The irrigation system according to claim 1, wherein the second material comprises a nitrile.

3. The irrigation system according to claim 1, wherein the second material comprises a heat shrink material.

4. The irrigation system according to claim 1, wherein the second material comprises a cotton fiber.

5. The irrigation system according to claim 1, wherein the second material comprises an ESD-preventing additive.

6. The irrigation system according to any one of claims 1 to 5, wherein the treatment (28) further resists abrasion of the tubular core (26) during operation of the peristaltic pump (10).

7. The irrigation system according to any one of claims 1 to 6, further comprising:
a first key (25) at a first end of the pump interface tubing (24); and
a second key (25) at a second end of the pump interface tubing (24),
wherein the first key (25) and the second key (25) are adapted to interconnect the pump interface tubing (24) with an irrigation tube (22).

8. The irrigation system according to any one of claims 1 to 7, wherein the treatment (28) extends along less than an entire length of the outer surface of the tubular core (26).

9. The irrigation system according to claim 1, wherein the second material comprises the first material of the tubular core (26) mixed with an ESD-preventing additive.

10. A method of manufacturing a reduced-noise pump interface tubing (24) for use in a peristaltic pump (10) of an irrigation system for use with an irrigated electrophysiology catheter, the method comprising forming a noise-reduction layer (28) about a tubular core (26), wherein forming a noise-reduction layer about a tubular core (26) preferably comprises co-extruding the tubular core (26) from a first material and the noise-reduction layer (28) from a second material different from the first material, wherein the second material is closer to zero on the triboelectric scale than the first material.

11. The method according to claim 10, wherein forming a noise-reduction layer (28) about a tubular core (26) comprises forming a nitrile layer about the tubular core (26), wherein forming a nitrile layer about the tubular core (26) preferably comprises dip-coating the nitrile layer about the tubular core (26), or forming a cotton fiber layer about the tubular core (26), or forming a heat shrink layer about the tubular core (26).

## Patentansprüche

1. Irrigationssystem zur Verwendung mit einem gespülten elektrophysiologischen Katheter, enthaltend:
eine Peristaltikpumpe (10); und
einen Pumpenschnittstellenschlauch (24) zur Verwendung in einer Peristaltikpumpe (10), wobei der Pumpenschnittstellenschlauch (24) enthält:
einen rohrförmigen Kern (26), der eine Außenfläche aufweist; und
eine Behandlung (28) auf der Außenfläche des rohrförmigen Kerns (26), wobei die Behandlung (28) ein statisches Aufladen des rohrförmigen Kerns (28) während eines Betriebs der Peristaltikpumpe (10) reduziert,
wobei der rohrförmige Kern (26) ein erstes Material enthält und wobei die Behandlung (28) eine Lage aus einem zweiten Material enthält, das sich von dem ersten Material unterscheidet, das um den rohrförmigen Kern (26) herum gebildet ist, und wobei das zweite Material auf der triboelektrischen Skala näher an Null ist als das erste Material.

2. Irrigationssystem nach Anspruch 1, wobei das zweite Material ein Nitril enthält.

3. Irrigationssystem nach Anspruch 1, wobei das zweite Material ein Wärmeschrumpfmaterial enthält.

4. Irrigationssystem nach Anspruch 1, wobei das zweite Material eine Baumwollfaser enthält.

5. Irrigationssystem nach Anspruch 1, wobei das zweite Material einen ESD-verhindernden Zusatz enthält.

6. Irrigationssystem nach einem der Ansprüche 1 bis 5, wobei die Behandlung (28) ferner einem Abrieb des rohrförmigen Kerns (26) während eines Betriebs der Peristaltikpumpe (10) widersteht.

7. Irrigationssystem nach einem der Ansprüche 1 bis 6, ferner enthaltend:
einen ersten Schlüssel (25) an einem ersten Ende des Pumpenschnittstellenschlauchs (24); und
einen zweiten Schlüssel (25) an einem zweiten Ende des Pumpenschnittstellenschlauchs (24),
wobei der erste Schlüssel (25) und der zweite Schlüssel (25) angepasst sind zum Verbinden des Pumpenschnittstellenschlauchs (24) mit einem Irrigationsschlauch (22).

8. Irrigationssystem nach einem der Ansprüche 1 bis 7, wobei die Behandlung (28) sich entlang von weniger als einer gesamten Länge der Außenfläche des rohrförmigen Kerns (26) erstreckt.

9. Irrigationssystem nach Anspruch 1, wobei das zweite Material das erste Material des rohrförmigen Kerns (26) gemischt mit einem ESD-verhindernden Zusatz enthält.

10. Verfahren zum Herstellen eines Geräusch-reduzierenden Pumpenschnittstellenschlauchs (24) zur Verwendung in einer Peristaltikpumpe (10) eines Irrigationssystems zur Verwendung mit einem gespülten elektrophysiologischen Katheter, wobei das Verfahren ein Ausbilden einer Geräuschreduktionsschicht (28) um einen rohrförmigen Kern (26) herum enthält, wobei das Ausbilden einer Geräuschreduktionsschicht um einen rohrförmigen Kern (26) herum vorzugsweise das Koextrudieren des rohrförmigen Kerns (26) aus einem ersten Material und der Geräuschreduktionsschicht (28) aus einem zweiten Material, das sich von dem ersten Material unterscheidet, enthält, wobei das zweite Material auf der triboelektrischen Skala näher an Null ist als das erste Material.

11. Verfahren nach Anspruch (10), wobei das Ausbilden einer Geräuschreduktionsschicht (28) um einen rohrförmigen Kern (26) herum ein Ausbilden einer Nitrilschicht um den rohrförmigen Kern (26) herum enthält, wobei das Ausbilden einer Nitrilschicht um den rohrförmigen Kern (26) herum vorzugsweise Tauchbeschichten der Nitrilschicht um den rohrförmigen Kern (26) herum oder Bilden einer Baumwollfaserschicht um den rohrförmigen Kern (26) herum oder Bilden einer Wärmeschrumpfschicht um den rohrförmigen Kern (26) herum enthält.

## Revendications

1. Système d'irrigation destiné à être utilisé avec un cathéter d'électrophysiologie irrigué, comprenant :
une pompe péristaltique (10) ; et
une tubulure d'interface de pompe (24) destinée à être utilisé dans une pompe péristaltique (10), la tubulure d'interface de pompe (24) comprenant :
un noyau tubulaire (26) ayant une surface extérieure ; et
un traitement (28) sur la surface extérieure du noyau tubulaire (26), dans lequel le traitement (28) réduit l'accumulation de charge statique sur le noyau tubulaire (28) pendant le fonctionnement de la pompe péristaltique (10),
dans lequel le noyau tubulaire (26) comprend un premier matériau et dans lequel le traitement (28) comprend une couche d'un second matériau différent du premier matériau formé autour du noyau tubulaire (26), et dans lequel le second matériau est plus proche de zéro sur l'échelle triboélectrique que le premier matériau.

2. Système d'irrigation selon la revendication 1, dans lequel le second matériau comprend un nitrile.

3. Système d'irrigation selon la revendication 1, dans lequel le second matériau comprend un matériau thermorétractable.

4. Système d'irrigation selon la revendication 1, dans lequel le second matériau comprend une fibre de coton.

5. Système d'irrigation selon la revendication 1, dans lequel le second matériau comprend un additif empêchant les décharges électrostatiques.

6. Système d'irrigation selon l'une quelconque des revendications 1 à 5, dans lequel le traitement (28) résiste en outre à l'abrasion du noyau tubulaire (26) pendant le fonctionnement de la pompe péristaltique (10).

7. Système d'irrigation selon l'une quelconque des revendications 1 à 6, comprenant en outre :
une première clé (25) à une première extrémité de la tubulure d'interface de pompe (24) ; et
une deuxième clé (25) à une deuxième extrémité de la tubulure d'interface de pompe (24),
dans lequel la première clé (25) et la seconde clé (25) sont adaptées pour interconnecter la tubulure d'interface de pompe (24) avec un tube d'irrigation (22).

8. Système d'irrigation selon l'une quelconque des revendications 1 à 7, dans lequel le traitement (28) s'étend sur moins de la longueur totale de la surface extérieure du noyau tubulaire (26).

9. Système d'irrigation selon la revendication 1, dans lequel le second matériau comprend le premier matériau du noyau tubulaire (26) mélangé avec un additif empêchant les décharges électrostatiques.

10. Procédé de fabrication d'une tubulure d'interface de pompe à bruit réduit (24) destiné à être utilisé dans une pompe péristaltique (10) d'un système d'irrigation destiné à être utilisé avec un cathéter d'électrophysiologie irrigué, le procédé comprenant la formation d'une couche de réduction de bruit (28) autour d'un noyau tubulaire (26), dans lequel la formation d'une couche de réduction de bruit autour d'un noyau tubulaire (26) comprend de préférence la co-extrusion du noyau tubulaire (26) à partir d'un premier matériau et de la couche de réduction de bruit (28) à partir d'un second matériau différent du premier matériau, dans lequel le second matériau est plus proche de zéro sur l'échelle triboélectrique que le premier matériau.

11. Procédé selon la revendication 10, dans lequel la formation d'une couche de réduction de bruit (28) autour d'un noyau tubulaire (26) comprend la formation d'une couche de nitrile autour du noyau tubulaire (26), dans lequel la formation d'une couche de nitrile autour du noyau tubulaire (26) comprend de préférence le revêtement par immersion de la couche de nitrile autour du noyau tubulaire (26), ou la formation d'une couche de fibre de coton autour du noyau tubulaire (26), ou la formation d'une couche thermorétractable autour du noyau tubulaire (26).
